# FASCICULE DE BREVET EUROPEEN

(11) **EP 2 807 486 B1**
(45) Date de publication et mention de la délivrance du brevet: **15.03.2017**
(21) Numéro de dépôt: 13704195.0
(22) Date de dépôt: 23.01.2013
(51) Int. Cl.: G01N 33/569

(54) **PROCEDE POUR PRONOSTIQUER IN VITRO DANS UN ECHANTILLON SANGUIN LA PROBABILITE POUR UN PATIENT D'EVOLUER VERS UNE DENGUE SEVERE**
VERFAHREN ZUR IN-VITRO-VORHERSAGE DER ENTWICKLUNG EINER SCHWEREN DENGUE-ERKRANKUNG EINES PATIENTEN AUF BASIS EINER BLUTPROBE
METHOD FOR THE IN VITRO PREDICTION OF THE PROBABILITY OF A PATIENT DEVELOPING SEVERE DENGUE, BASED ON BLOOD SAMPLE

(30) Priorité: 24.01.2012 FR 1250646
(43) Date de publication de la demande: 03.12.2014
(73) Titulaire: Biomérieux, 69280 Marcy l'Étoile (FR)
(72) Inventeur: BEDIN, Frédéric, 69007 Lyon (FR)
(86) Numéro de dépôt international: PCT/FR2013/050143
(87) Numéro de publication internationale: WO 2013/110894

(56) Documents cités:
- WO-A1-2011/092219
- WO-A2-2009/145810
- PAWITAN JEANNE A: "Dengue virus infection: predictors for severe dengue.", ACTA MEDICA INDONESIANA APR 2011, vol. 43, no. 2, avril 2011 (2011-04), pages 129-135, XP002688549, ISSN: 0125-9326
- SRIKIATKHACHORN ANON ET AL: "Markers of dengue disease severity.", CURRENT TOPICS IN MICROBIOLOGY AND IMMUNOLOGY 2010, vol. 338, 2010, pages 67-82, XP009165400, ISSN: 0070-217X
- LIDIANE M ALBUQUERQUE ET AL: "Two-Dimensional Difference Gel Electrophoresis (DiGE) Analysis of Plasmas from Dengue Fever Patients", JOURNAL OF PROTEOME RESEARCH, AMERICAN CHEMICAL SOCIETY, WASHINGTON, DC, US, vol. 8, no. 12, 4 décembre 2009 (2009-12-04), pages 5431-5439, XP008146903, ISSN: 1535-3907, DOI: 10.1021/PR900236F [extrait le 2009-10-21]
- CHAIYARATANA WATHANEE ET AL: "Serum ferritin levels in children with dengue infection", SOUTHEAST ASIAN JOURNAL OF TROPICAL MEDICINE AND PUBLIC HEALTH, PROJECT OD SEAMEO, BANGKOK, vol. 39, no. 5, 1 septembre 2008 (2008-09-01), pages 832-836, XP009165364, ISSN: 0125-1562
- WEIVODA S ET AL: "ELISA for human serum leucine-rich alpha-2-glycoprotein-1 employing cytochrome c as the capturing ligand", JOURNAL OF IMMUNOLOGICAL METHODS, ELSEVIER SCIENCE PUBLISHERS B.V.,AMSTERDAM, NL, vol. 336, no. 1, 20 juillet 2008 (2008-07-20), pages 22-29, XP022682287, ISSN: 0022-1759, DOI: 10.1016/J.JIM.2008.03.004 [extrait le 2008-04-04]
- ROMAIN FRAGNOUD ET AL: "Isotope Coded Protein Labeling analysis of plasma specimens from acute severe dengue fever patients", PROTEOME SCIENCE, BIOMED CENTRAL, LONDON, GB, vol. 10, no. 1, 26 octobre 2012 (2012-10-26), page 60, XP021136424, ISSN: 1477-5956, DOI: 10.1186/1477-5956-10-60

## Description

La présente invention a pour objet un procédé pour le pronostic précoce d'une dengue sévère ou dengue hémorragique utilisant des marqueurs protéiques.

Ces 30 dernières années, la dengue, une maladie virale transmise par les moustiques hématophages urbains du genre *Aedes*, s'est répandue à travers le monde de manière inquiétante. C'est actuellement un véritable problème de santé publique pour plus d'une centaine de pays situés dans la zone subtropicale, particulièrement dans les zones pacifique-ouest, Amérique du sud et Asie du sud-est. L'émergence de la maladie est due en grande partie à l'explosion démographique et à l'urbanisation anarchique. Les anomalies climatiques ont également un rôle non négligeable. A ce titre, la dengue pourrait émerger dans les régions occidentales du globe jusqu'alors épargnées par le virus. Ainsi, *Aedes albopictus,* un des vecteurs de la maladie, a récemment été trouvé dans le nord de l'Italie et dans le sud de la France. Dernièrement, des cas de dengue autochtones ont été signalés dans le sud de la France. On estime que près de 3 milliards de personnes sont exposés aux risques de dengue. Près d'un million d'hospitalisations sont recensées annuellement et les décès se comptent par milliers. Les enfants sont les principales victimes de la maladie. Le virus de la dengue est un virus enveloppé à ARN monocaténaire de polarité positive de la famille des Flaviviridae. Le génome du virus (11 000 nucléotides) code pour une polyprotéine d'environ 3400 acides aminés qui subit un clivage co- et post-traductionnel qui résulte en des protéines structurales (C, prM, E) et des protéines non-structurales (NS1, NS2A, NS2B, NS3, NS4A, NS4B, NS5). Il existe 4 sérotypes viraux (DV1 à DV4), qui peuvent coexister en zones d'endémie. Il y a environ 70% d'homologie de séquences entre les différents sérotypes. L'infection par un sérotype donné confère une immunité sur le long terme pour ce sérotype. La protection croisée ne dure que quelques mois : la réinfection est donc possible avec un sérotype différent. La manifestation clinique la plus commune (fièvre de dengue « classique » ou *Dengue Fever* : DF) est un état fébrile de quelques jours accompagné notamment de céphalées sévères, de lombalgies, de douleurs musculaires et articulaires, qui régressent spontanément sans traitement spécifique au bout de quelques jours. Cependant, on assiste parfois à des complications conduisant à une dengue hémorragique (*Dengue Hemorhagic Fever* : DHF). Dans ce cas, on note une augmentation transitoire de la perméabilité vasculaire et une fuite du plasma responsable d'une thrombopénie et d'une coagulopathie. Dans les cas les plus sévères, la fuite de plasma peut entraîner un choc hypovolumique mortel (*Dengue Shock Syndrome* : DSS) si le patient n'est pas pris en charge rapidement. Des atteintes hépatiques et neurologiques, rares mais mortelles, sont également associées à la sévérité de la maladie. Variable selon les épidémies, le taux de mortalité peut atteindre 5% des cas déclarés de DHF. Ce taux peut augmenter jusqu'à 20% sans une prise en charge hospitalière ou des traitements adaptés.

90% des cas de DHF ont lieu lors d'une infection secondaire par un sérotype hétérologue et 10% lors d'une infection primaire, habituellement chez des nourrissons âgés de 6 mois à 1 an. Il existe plusieurs facteurs qui influent sur la sévérité de l'infection, tels que les facteurs de l'hôte, le sérotype et le génotype du virus, l'ordre de succession des virus infectants, la qualité et la quantité d'anticorps à réactions croisées et la réponse CD4/CD8. Les causes exactes de l'apparition de la DHF ne sont cependant toujours pas connues avec certitude. Dans une première hypothèse, ce sont les forces de sélection exercées sur le virus qui conduisent à la sélection d'un « super-virus ». Des études ont montré une corrélation entre la charge virale et la sévérité de la maladie. Des études ont également impliqué les protéines virales E et NS1 dans la pathogénicité. La séquence des sérotypes infectants, l'intervalle entre chaque infection sont également des déterminants cliniques importants.

Une infection secondaire avec un sérotype différent est souvent associée à la sévérité de la maladie, mais n'est pas un facteur de certitude. C'est la base de la deuxième hypothèse, l'hypothèse de la facilitation de l'infection par des anticorps anti-dengue de faible affinité, qui n'a jamais pu être confirmée *in vivo* en l'absence de modèle animal. Elle repose sur la présence d'anticorps neutralisants de faible affinité qui facilitent l'infection *in vitro* des macrophages via leur récepteur Fc aux immunoglobulines. La présence des complexes immuns favorise par ailleurs l'activation du complément.

L'activation et la prolifération excessive des lymphocytes T CD4 et CD8+ mémoires entraîneraient la production accrue de cytokines et de médiateurs cellulaires. La concomitance de ces différents éléments pourrait être responsable de la pathogénie. Si la mise en évidence du rôle clef de l'activation lymphocytaire et de la sécrétion de médiateurs dans la physiopathologie est assez bien admise, il est très difficile de montrer une corrélation claire avec l'apparition de la DHF.

Les facteurs génétiques jouent probablement un rôle puisque plusieurs études ont montrées le rôle soit protecteur soit pathogène de certains allèles de HLA classe 1. Cependant, les résultats sont assez contradictoires et varient suivant l'origine des prélèvements.

Jusqu'à présent, aucun déterminant spécifique de la virulence n'a été mis en évidence avec certitude. De plus, comme il n'existe pas de vaccins contre le virus de la dengue, les seuls traitements disponibles sont des traitements symptomatiques. C'est pourquoi, il est important de pouvoir surveiller les épidémies et de pronostiquer les cas sévères pour une prise en charge hospitalière adaptée [1, 2, 3].

De nombreux marqueurs pour le pronostic de l'évolution vers une dengue sévère ont déjà été décrits dans la littérature scientifique. Ainsi, Pawitan Jeanne A [5] décrit l'évaluation de la charge virale, l'utilisation de cytokines, d'interférons, de facteurs tumoraux, d'interleukines ou encore de la protéine NS1 comme marqueur biologique de la prédiction de la dengue sévère. Srikiatkhachorn A et al., 2010 [6] divulguent également l'utilisation de cytokines (telles que sCD4, sCD8, IL-1Ra...), de facteur de croissance de l'endothélium vasculaire (VEGF), ou encore de la protéine NS1 en tant que marqueurs précoces d'évolution vers une dengue sévère.

En 2009, Lidiane M Albuquerque et al. [7] décrivent l'utilisation de plusieurs protéines dont la DBP (vitamin D-binding protein), la FGG (fibrinogen gamma chain), l'Apolipoprotréine J ou encore le Complément C3 en tant que biomarqueurs pour le diagnostic, le pronostic ou encore le traitement de la dengue.

La publication de Chaiyaratana Wathanee et al. [8] propose, quant à elle, d'étudier le niveau de ferritine dans le sérum d'enfants atteints de la dengue en phase aiguë dans le but de prédire le risque de contracter une dengue hémorragique.

La demande de brevet WO 2009/145810 décrit, quant à elle, l'utilisation d'un membre de la famille des récepteurs de la cytokine (ST2) pour le diagnostic de la dengue sévère et non pour le pronostic de la probabilité d'évoluer vers une dengue sévère.

L'utilisation de la LGR a déjà été décrite mais pour le diagnostic de maladies différentes. Ainsi, la demande de brevet WO 2011/092219 décrit l'utilisation de la LRG en tant que biomarqueur pour le diagnostic des maladies cardio-vasculaires, plus particulièrement des dysfonctionnements ventriculaires et insuffisances cardiaques et pour la prédiction des arrêts cardiaques. La publication de Weivoda S et al. (2008) [9] décrit également une étude sur le taux de glycoprotéine LRG dans le sérum de patients atteints d'infection bactérienne, de SIDA, d'arthrite, de troubles neurologiques ou encore de Parkinson.

Les méthodes actuellement utilisées pour le diagnostic de la dengue ne permettent pas de pronostiquer une évolution vers une dengue sévère. Tout au plus, les méthodes sérologiques permettent de discriminer infections primaires et secondaires et les méthodes moléculaires permettent de détecter le virus et d'effectuer le sérotypage.

La présente invention répond aux problématiques présentées ci-dessus par un procédé qui permet à la fois une détection précoce et spécifique de protéines dans un échantillon sanguin permettant de pronostiquer les cas de dengues sévères (DHF et/ou DSS). En effet, la Demanderesse a trouvé que des protéines étaient quantitativement plus abondantes ou surexprimées dans les cas de dengues sévères en comparaison avec leur quantité ou expression dans des cas de dengues classiques dans des échantillons sanguins correspondant à du sang total, du sérum ou du plasma. Tout particulièrement, ils ont montré pour la première fois et de manière totalement inattendue que la Leucine-riche alpha-2 glycoprotéine (LRG) est surexprimée dans le cas des DHF et qu'elle constitue donc un marqueur de pronostic de la dengue sévère.

Aussi la présente invention a pour objet un procédé pour pronostiquer, *in vitro,* dans un échantillon sanguin, la probabilité pour un patient d'évoluer vers une dengue sévère, dans lequel :
a)on détermine la quantité d'au moins un marqueur qui est la Leucine-riche alpha-2 glycoprotéine dans ledit échantillon sanguin,
b)on compare la quantité de la leucine-riche alpha-2 glycoprotéine déterminée dans l'étape a) avec une quantité de référence dudit marqueur obtenue à partir d'un groupe d'individus qui ont été diagnostiqués comme étant atteints d'une dengue non sévère,
dans lequel si la quantité de la leucine-riche alpha-2 glycoprotéine déterminée dans l'étape a) est supérieure à la quantité de référence établie dans l'étape b), on pronostique que le patient va évoluer vers une dengue sévère.

Dans un mode de réalisation du procédé de l'invention dans l'étape a) on détermine de plus la quantité d'au moins un autre marqueur choisi parmi la Vitamine D Binding-Protéine et la Ferritine dans l'échantillon sanguin et dans l'étape b) on compare la quantité dudit autre marqueur de l'étape a) avec une quantité de référence dudit autre marqueur obtenue à partir d'un groupe d'individus qui ont été diagnostiqués comme étant atteints d'une dengue non sévère et si la quantité dudit au moins autre marqueur déterminée dans l'étape a) est supérieure à la quantité de référence établie dans l'étape b), on pronostique que le patient va évoluer vers une dengue sévère.

Dans un autre mode de réalisation du procédé de l'invention dans l'étape a) on détermine de plus la quantité d'au moins deux autres marqueurs choisi parmi la Vitamine D Binding-Protéine et la Ferritine dans l'échantillon sanguin et dans l'étape b) on compare la quantité de chacun des deux autres marqueurs de l'étape a) avec une quantité de référence pour chacun desdits autres marqueurs obtenue à partir d'un groupe d'individus qui ont été diagnostiqués comme étant atteints d'une dengue non sévère et si la quantité de chacun des autres marqueurs déterminée dans l'étape a) est supérieure à la quantité de référence de chacun des marqueurs établie dans l'étape b), on pronostique que le patient va évoluer vers une dengue sévère.

L'invention a aussi pour objet l'utilisation pour le pronostic *in vitro* d'une dengue sévère chez un patient de :
- la Leucine-riche alpha-2 glycoprotéine comme marqueur de la pathologie,
- la Leucine-riche alpha-2 glycoprotéine et de la Vitamine D Binding Protéine ou de la Ferritine comme marqueurs de la pathologie, et
- de la Leucine-riche alpha-2 glycoprotéine et de la Vitamine D Binding Protéine et de la Ferritine comme marqueurs de la pathologie.

L'invention concerne également un kit pour le pronostic *in vitro* d'une dengue sévère comprenant :
- un partenaire de liaison de la Leucine-riche alpha-2 glycoprotéine,
- un partenaire de liaison de la Vitamine D Binding Protéine, et
- un partenaire de liaison de la Ferritine, plus facultativement
- au moins un partenaire de liaison d'au moins une protéine du virus de la dengue choisie parmi la protéine NS1, la protéine d'enveloppe et la protéine prM.

### Définitions

Par échantillon sanguin on entend le sang total, le sérum et le plasma.

Par partenaire de liaison on entend par exemple les récepteurs, les anticorps, les fragments d'anticorps, les analogues d'anticorps et tout autre ligand capable de se lier à une protéine.
Les anticorps partenaires de liaison sont par exemple soit des anticorps polyclonaux, soit des anticorps monoclonaux.
Les anticorps polyclonaux peuvent être obtenus par immunisation d'un animal avec l'immunogène approprié, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixé un antigène spécifiquement reconnu par les anticorps.

Les anticorps monoclonaux peuvent être obtenus par la technique des hybridomes dont le principe général est rappelé ci-dessous.

Dans un premier temps, on immunise un animal, généralement une souris avec l'immunogène approprié, dont les lymphocytes B sont alors capables de produire des anticorps contre cet antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis de la protéine pourront être testées par exemple en ELISA, par immunotransfert (Western blot) en une ou deux dimensions, en immunofluorescence, ou à l'aide d'un biocapteur. Les anticorps monoclonaux ainsi sélectionnés, sont par la suite purifiés notamment selon la technique de chromatographie d'affinité décrite ci-dessus.

Les anticorps monoclonaux peuvent être également des anticorps recombinants obtenus par génie génétique, par des techniques bien connues de l'homme du métier.

Par « analogues d'anticorps » on entend des composés biologiques et/ou chimiques qui possèdent les mêmes capacités de liaison que les anticorps ou fragments d'anticorps ou des capacités de liaison similaires. En particulier, les analogues d'anticorps incluent de petites protéines qui comme les anticorps sont capables de se lier à une cible biologique permettant ainsi de la détecter, de la capturer ou tout simplement de la cibler au sein d'un organisme ou d'un échantillon biologique. Les champs d'applications de ces analogues d'anticorps sont pratiquement aussi vastes que ceux des anticorps. A titre d'exemple, on peut citer les Nanofitines^{™}, petites protéines commercialisées par la société AFFILOGIC.

Les partenaires de liaison spécifiques de la protéine recherchée peuvent être utilisés comme réactif de capture, comme réactif de détection ou comme réactifs de capture et de détection.

La visualisation des réactions immunologiques, c'est-à-dire de la liaison protéine/partenaire de liaison, peut être effectuée par tout moyen de détection par l'intermédiaire d'un marquage, du partenaire de liaison.

Par marquage, on entend la fixation d'un réactif marqueur capable de générer un signal détectable, c'est à dire un composé, une substance ou une particule qui peut être détecté par des moyens visuels, fluorescents, instrumentaux.

Une liste non limitative de ces réactifs marqueurs consiste en :
- les particules métalliques ou d'alliages, telles que les particules d'or colloïdal,
- les particules de polymère, telles que les particules de latex colorés,
- les particules magnétiques,
- les molécules fluorescentes,
- les molécules chimioluminescentes.
A titre d'exemple de tests immunologiques tels que définis ci-dessus, on peut citer les méthodes « sandwich » et de « compétition ».

### Figures :

Les figures illustrent la confirmation et la validation des résultats d'ICPL pour chaque marqueur candidat sélectionné par un test ELISA quantitatif effectué sur des échantillons individuels de patients prélevés durant la phase aigüe de la maladie (patients DF et DHF), avant défervescence. Dans tous les cas la lecture est effectuée à une densité optique (DO) de 450nm. Les résultats ont été obtenus sur les échantillons tahitiens, sur les échantillon colombiens et sur un mélange des deux. La moyenne calculée est représentée par un trait horizontal (Logiciel GraphPad Prism). Les valeurs correspondent à deux essais indépendants effectués en duplicate.
La figure 1 illustre les résultats obtenus pour le dosage quantitatif par test ELISA du marqueur LRG sur des échantillons de plasma provenant de patients tahitiens.
La figure 2 illustre les résultats obtenus pour le dosage quantitatif par test ELISA du marqueur LRG sur des échantillons de sérum provenant de patients colombiens.
La figure 3 illustre les résultats obtenus pour le dosage quantitatif par test ELISA du marqueur ferritine sur des échantillons de plasma provenant de patients tahitiens.
La figure 4 illustre les résultats obtenus pour le dosage quantitatif par test ELISA du marqueur ferritine sur des échantillons de sérum provenant de patients colombiens.
La figure 5 illustre les résultats obtenus pour le dosage quantitatif par test ELISA du marqueur ferritine sur un mélange de plasmas d'origine tahitienne et de sérums d'origine colombienne.
La figure 6 illustre les résultats obtenus pour le dosage quantitatif par test ELISA du marqueur CRP sur des échantillons de plasma provenant de patients tahitiens.
La figure 7 illustre les résultats obtenus pour le dosage quantitatif par test ELISA du marqueur CRP sur des échantillons de sérum provenant de patients colombiens.
La figure 8 illustre les résultats obtenus pour le dosage quantitatif par test ELISA du marqueur Galectine3 Binding protein sur des échantillons de plasma provenant de patients tahitiens.
La figure 9 illustre les résultats obtenus pour le dosage quantitatif par test ELISA du marqueur Vitamine D Binding Protein sur un mélange de plasmas d'origine tahitienne et de sérums d'origine colombienne.
La figure 10 illustre les résultats obtenus pour le dosage quantitatif par test ELISA du marqueur Afamine sur des échantillons de plasma provenant de patients tahitiens.

### Exemples

### Exemple 1 : Sélection et pré-traitement des échantillons

10 échantillons de plasma de patients provenant d'une étude rétrospective menée par l'Institut Louis Malardé, Papeete, Tahiti (Polynésie Française) ont été sélectionnés, parmi lesquels 5 échantillons de patients ayant développé une dengue classique (DF) et 5 échantillons de patients ayant développé une dengue sévère (DHF) qui ont été regroupés constituant ainsi un pool DF et un pool DHF. Les critères de sélection étaient une homogénéité en âge (llans +/- 1 an) et date de prélèvement après l'apparition des symptômes (4 jours +/- 1 jour). En effet, un marqueur pronostique des formes sévères doit être détectable après l'apparition des symptômes de l'infection par le virus de la dengue (apparition de la fièvre) mais avant la défervescence, qui correspond au passage vers les formes sévères. La proportion homme/femme était identique (rapport=3/2). Dans les deux pools, les patients étaient atteints d'une infection secondaire par un virus de sérotype 2 (DV2). Le taux d'immunoglobuline M spécifiques était faible ou indétectable. La charge virale était détectable dans tous les cas. Pour chaque pool, 40 µL de chacun des 5 échantillons ont été mélangés ce qui constitue 2 groupes d'échantillons DF et DHF d'un volume total de 200µL.
Afin d'exclure les protéines les plus représentées (albumine, immunoglobuline, transférine, fibrinogène, alpha-2 microglobuline, haptoglobuline, etc...) dont la présence peut masquer les protéines faiblement représentées et donc induire un biais au niveau du choix des protéines d'intérêt, une déplétion a été réalisée grâce au kit Proteo-prep20™ commercialisé par Sigma-Aldrich (USA). Ce kit permet une déplétion des 20 protéines plasmatiques les plus abondantes. Le protocole utilisé a été celui préconisé par le fournisseur. A l'issue de cette immuno-déplétion, 2 groupes d'échantillons étaient disponibles : un pool DF déplété et un pool DHF déplété. Les pools ont été contrôlés sur gel de polyacrylamide 4-12% Bis-tris (InVitrogen™, UK) afin de valider l'étape de déplétion.

### Exemple 2 : Identification des protéines spécifiques de chaque pool

Après, réduction et alkylation des résidus Cystéines, chaque pool a été analysé de manière différentielle par la technique d'ICPL (*Isotope Coded Labelled Protein*)*,* développée par la société Brucker™.

Cette technique permet les études différentielles par marquage des échantillons à comparer [4]. Les protéines sont marquées sur leur Lysines de façon spécifique avec un réactif contenant du ¹²C : isotope léger pour le pool DF et avec un réactif contenant du ¹³C : isotope lourd pour le pool DHF. Il en résulte une différence de masse, apportée par les deux isotopes. Après mélange, des deux échantillons marqués différemment, les protéines sont fractionnées par électrophorèse sur gel de polyacrylamide en 1 dimension. Le gel est alors fractionné en 20 bandes. Ces bandes sont hydrolysées par de la trypsine. Les protéines de chaque hydrolysat sont séparées par chromatographie liquide NANOLC, ionisées par ionisation par electrospray et identifiées par spectrométrie de masse avec trappe ionique (2 dépôts par bande). Le logiciel WARPLC permet une sélection automatique des pics différentiels à identifier par MS/MS. Le tableau ci-dessous liste les protéines candidates identifiées à l'issue de cette étude. Le différentiel protéique identifiée par ICPL sur les pool DF et DHF est détaillé. Le tableau indique la nature de la protéine et le rapport de signal isotope lourd/isotope léger *(Avg.).* Pour certaines protéines, le rapport lourd/léger est en faveur d'une forte proportion de signal « léger » (Avg. < 0.5), donc chaque protéine est potentiellement un marqueur spécifique des dengues classiques, non-sévères (DF). Pour d'autres protéines, le rapport lourd/léger est en faveur d'une forte proportion de signal « lourd » (Avg. > 1.6) et chaque protéine est potentiellement un marqueur spécifique des dengues sévères. Les inventeurs ont sélectionné des protéines comme candidats marqueurs potentiels de la DF ou DHF. Il s'agit de :
- La péroxyrédoxine-2 (peroxyredoxin-2),
- La protéine de liaison de la vitamine D (vitamin D-binding protein),
- L'afamine (Afamin),
- L'alpha-2 glycoprotein riche en Leucine (Leucin rich alpha-2 glycoprotein),
- La protéine de liaison de la Galectine-3 (Galectin-3 binding protein),
- La protéine C-réactive (C-reactiv protein),
- La chaîne légère de la ferritine (Ferritin light chain)

L'haptoglobine a été exclue car elle fait partie des protéines majoritaires du plasma et aurait dû normalement être éliminée par la colonne Proteoprep20. La protéine du complément C7 n'a pas non plus été retenue du fait de son caractère ubiquitaire.

**Tableau 1 : protéines identifiées par ICPL sur les pools de plasmas de Tahiti**

| Protéine | Accession | H/L | Avg | Max. Avg | Max.cv |
|---|---|---|---|---|---|
| Peroxyredoxine 2 | P322119 | 9 | 0,33 | 0,08 | 41,55 |
| Haptoglobine | P00738 | 13 | 0,43 | 0,150 | 45,03 |
| complément C7 | P10643 | 8 | 0,48 | 0,030 | 12,026 |
| Apolipoproteine A | P06727 | 3 | 0,68 | 0,310 | 48,72 |
| Fibrinogene chaine Alfa | P02671 | 4 | 0,79 | 0,030 | 5,16 |
| Apolipoprotéine E | P02649 | 6 | 0,8 | 0,0140 | 26,45 |
| Protéine A amiloïde | P02735 | 7 | 0,92 | 0,070 | 16,59 |
| Fibrinogene chaine béta | P002675 | 9 | 0,93 | 0,050 | 10,68 |
| Inter alpha-trypsine inhibiteur H1 | P19827 | 7 | 0,94 | 0,230 | 37,76 |
| Complément facteur H protéine 1 | Q03591 | 14 | 0,95 | 0,140 | 21,99 |
| Inter alpha-trypsine inhibiteur H2 | P19823 | 15 | 0,98 | 0,27 | 39,51 |
| Fibrinogène chaine gamma | P02679 | 3 | 0,99 | 0,01 | 1,87 |
| Vitronectine | P04004 | 2 | 1 | 0,1 | 14,28 |
| Complément facteur B | P00751 | 8 | 1,06 | 0,1 | 17,54 |
| Complémént C4 | POCOL4 | 3 | 1,06 | 0,304 | 9,24 |
| Albumine | P02768 | 27 | 1,09 | 0,18 | 25,57 |
| alpha-2 antiplasmine | P08697 | 5 | 1,18 | 0,203 | 4,13 |
| Antithrombine III | P01008 | 16 | 1,23 | 0,13 | 24,64 |
| Apolipoprotéine A1 | P02647 | 74 | 1,24 | 0,21 | 69,85 |
| Rétinol binding Protéine | P02753 | 5 | 1,24 | 0,13 | 13,3 |
| Prothrombine | P00734 | 4 | 1,26 | 0,15 | 14,18 |
| Complément facteur 1 | P05156 | 3 | 1,27 | 0,02 | 2,96 |
| Beta-microglobuline | P61769 | 3 | 1,27 | 0,05 | 4,94 |
| Ig alpha-1 chaine C | P01876 | 2 | 1,27 | 0,042 | 0,18 |
| Hemopexine | P02790 | 28 | 1,27 | 0,14 | 37,26 |
| Alpha 1B glycoprotéine | P04217 | 4 | 1,28 | 0,08 | 9,49 |
| Amiloide A4 protéine | P35542 | 6 | 1,29 | 0,14 | 16,83 |
| Kininogène 1 | P01042 | 3 | 1,3 | 0,346 | 11,97 |
| IgMu chaine C | P01871 | 2 | 1,3 | 0,057 | 0,32 |
| Cystéine-rich proteine 2 | P16562 | 2 | 1,3 | 0,212 | 4,5 |
| Zinc alpha-2glycoprotéine | P25311 | 7 | 1,3 | 0,12 | 19,75 |
| Extracellulaire matrice protéine | Q16610 | 2 | 1,32 | 0,057 | 0,32 |
| facteur épithéliaire pigmentaire | P36955 | 3 | 1,35 | 0,1 | 15,72 |
| Complément C3 | P01024 | 7 | 1,37 | 0,19 | 25,84 |
| Attractine | 075882 | 4 | 1,38 | 0,2 | 22,01 |
| Complémént-facteur H protéine 2 | P36980 | 2 | 1,41 | 1,443 | 208,08 |
| Apolipoprotéine B-100 | P04114 | 6 | 1,43 | 0,26 | 32,29 |
| Complément facteur H | P08603 | 24 | 1,46 | 0,41 | 29,06 |
| Alpha-1 Antichemoptrypsine | P01011 | 6 | 1,48 | 0,2 | 21,84 |
| Angiotensinogène | P01019 | 4 | 1,54 | 0,25 | 22,71 |
| Histidine-riche glycoprotéine | P04196 | 2 | 1,54 | 1,259 | 158,42 |
| Inter-alpha trypsine inhibiteur chaine H4 | Q14624 | 5 | 1,54 | 0,09 | 7,18 |
| Composant serum-amyloïde P | P02743 | 15 | 1,56 | 0,23 | 29,2 |
| Clusterine | P10909 | 7 | 1,57 | 0,25 | 29,73 |
| Vitamine D Binding-protéine | P02774 | 13 | 1,63 | 0,75 | 64,18 |
| Afamine | P43652 | 6 | 1,85 | 0,34 | 29,98 |
| Fibronectine | P02751 | 9 | 1,99 | 0,28 | 27,97 |
| Leucine -riche alpha-2 glycoprotéine | P02750 | 2 | 2,26 | 0,884 | 78,12 |
| Galectine -3 binding-protéine | Q08380 | 5 | 2,66 | 0,26 | 10,97 |
| Protéine C-réactive | P02741 | 3 | 3,87 | 0,365 | 13,29 |
| Ferritine chaine légère | P02792 | 5 | 5,75 | 1,7 | 39,29 |

### Exemple 3 : ELISA de confirmation

### Matériel et méthodes :

Afin de confirmer et valider les résultats d'ICPL, chaque marqueur-candidat a été testé en ELISA quantitatif sur des échantillons individuels. Ces échantillons étaient des échantillons de patients ayant développé soit une dengue « classique » , soit une dengue sévère et prélevés durant la phase aigüe de la maladie (phase virémique). Tous les patients avaient une dengue secondaire. Seuls les sérotypes 1, 2 et 3 étaient représentés (pas de sérotype 4) . Ces prélèvements étaient originaire de L'institut Louis Malardé (Tahiti, Polynésies Françaises) ou originaire de *l'Universidad Industrial de Santander* (Bucaramanga, Colombie). Ces derniers faisaient partie d'une étude rétrospective menée en accord avec le comité éthique local.

Les ELISA quantitatifs ont été effectués grâce à l'utilisation de kits commerciaux, en suivant les instructions fournies par les fabricants. Tous les échantillons ont été testés deux fois et en duplicate. La liste des kits commerciaux utilisés est la suivante : Pour la Vitamine D Binding-protéine : le kit Human DBP™, USCN Life (Wuhan, Chine),
pour l'Afamine : le kit AFM™, USCN life (Wuhan, Chine),
pour la Leucine-riche alpha-2 glycoprotéine : le kit Human-LRG™, IBL International (Hamburg, Allemagne),
pour la Galectine-3 binding-protéine . le kit Mac-2 BP™, IBL International (Hamburg, Allemagne),
pour la proteine C-réactive : le kit high-sensitive CRP ELISA™, IBL International (Hamburg, Allemagne)
pour la chaine légère de la Ferritine : le kit Ferritine™, IBL International (Hamburg, Allemagne).

Pour la Peroxyredoxine-2 , l'absence de tests commerciaux a nécessité la mise au point un ELISA. Pour ce faire, des anticorps polyclonaux anti-péroxyredoxine-2 obtenus soit chez le lapin (SAB2101878, Sigma-Aldrich USA) soit chez la chèvre (SAB2500777, Sigma-Aldrich) ont été utilisés. Ces anticorps sont dilués à 2µg/mL dans un tampon Tris-Maléate, pH6.2 2 et utilisés en fond de plaque. Après lavage, les plaques sensibilisées par ces anticorps sont saturées 1h à 37°C avec un tampon PBS-0.5% gélatine. L'échantillon à tester est alors ajouté à différentes dilutions dans du PBS-0.05%Tween20-0.1% gélatine et incubé 1h à 37°C. Après trois lavages au PBS-0.5% Tween20, un anticorps monoclonal de souris anti-peroxyredoxine-2 est ajouté (WHO007001M Sigma-Aldrich ; dilution :1µg/ml) et incubé 1h à 37°C. La révélation est effectuée à l'aide du kit One-Step NBT-BCIP™ (Thermo Scientific, USA) en présence d'un conjugué anti-espèce marqué à la phosphatase alcaline dilué à 0.1µg/mL (Jackson , USA).

### Résultats :

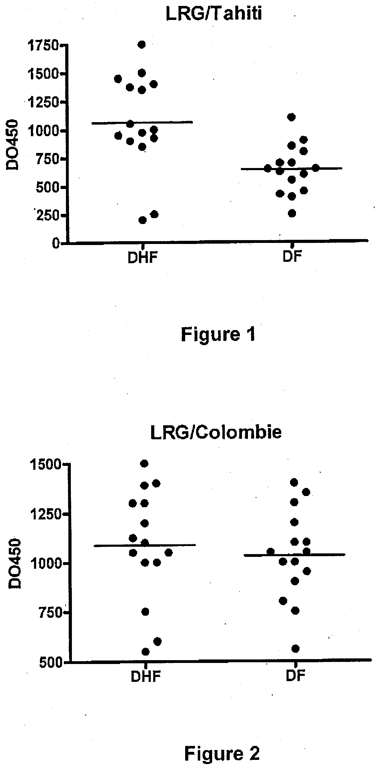

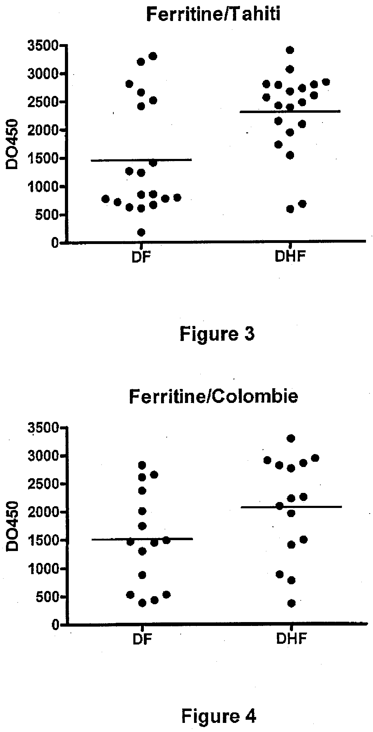

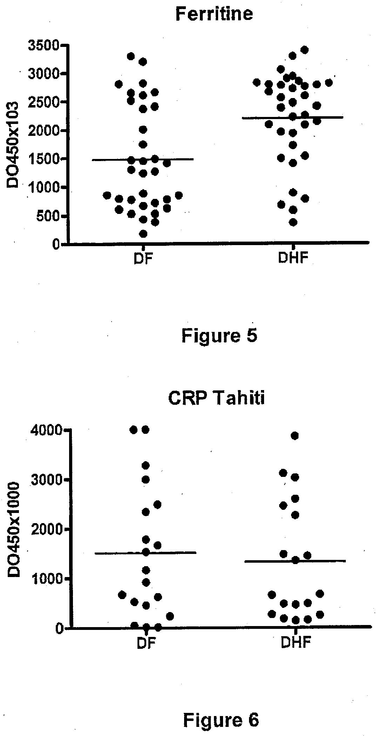

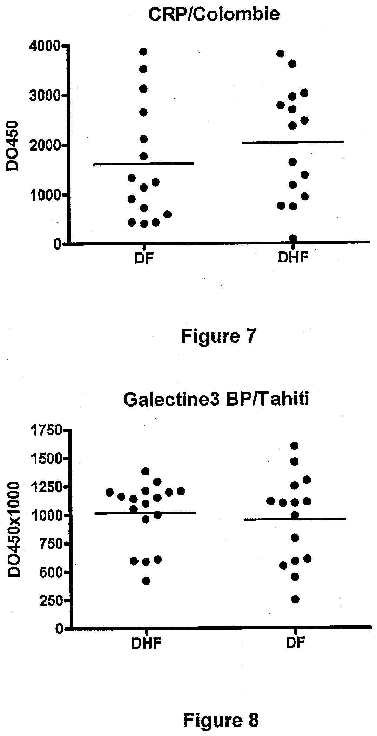

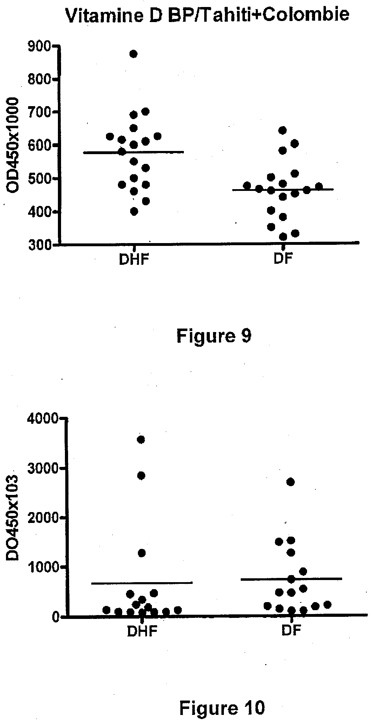

Les résultats sont détaillés dans les Figures 1 à 10 et dans le tableau 2 qui suit :

**Tableau 2 : valeurs de p pour les différents marqueurs candidats testés sur des échantillons tahitiens et/ou colombiens**

| | **Valeur de p** | | |
|---|---|---|---|
| **Marqueurs** | Colombie (sérum) | Tahiti (plasma) | Mélange |
| VitamineD BP | 0,01 | 0,008 | 0,0017 |
| LRG | 0,1 | 0,015 | 0,072 |
| Ferritine | 0,07 | 0,005 | 0,0018 |
| CRP | 0,1 | 0,4 | NT |
| Afamine | NT | 0,7 | NT |
| Peroxyredoxine2 | NT | 0,4 | 0,38 |
| G3BP | NT | 0,6 | NT |

Les différences obtenues entre les deux populations (DF/DHF) ont été validées par un test statistique (*Unpaired t-test,* logiciel Prism V4.03 Graphpad). Le seuil de significativité p<0,05 a été retenu, correspondant à un intervalle de confiance de 95%. les résultats montrent une quantité significativement supérieure dans les échantillons DHF pour les protéines suivantes : VitamineD Binding Protein (VitaminD BP), Leucine-riche glycoprotéine (LRG) et ferritine ce qui en fait des marqueurs très intéressants, pris individuellement ou en association, pour le pronostic d'une dengue sévère sur des échantillons sanguins dès l'apparition des premiers symptômes de la dengue.

### REFERENCES BIBLIOGRAPHIQUES

1. SB Halstead . The lancet 2007; 370: 1644-52
2. AS Leong et al. Semin.Diagn.Pathol. 2007; 24(4):227-236
3. K. Clyde et al. J. Virol. 2006; 23: 11418-11431
4. Lottspeich F, Kellermann J. ICPL labeling stratégies for proteome research. Methods Mol Biol. 2011;753:55-64
5. Pawitan Jeanne A. Acta Medica Indonesiana. 2011; 43(2):129-135
6. Srikiatkhachorn A et al., 2010. Current Topics in Microbiology and Immunology. 2010; vol.338: 67-82
7. Lidiane M Albuquerque et al. Journal of Proteome Research. American Chemical Society, Washington, DC, US. 2009; 8(12):5431-5439
8. Chaiyaratana Wathanee et al.Southeast Asian Journal of Tropical Medicine and Public Health, Project Od Seameo, Bangkok. 2008; 39(5):832-836
9. Weivoda S et al. Journal of Immunological Methods. 2008; 336(1): 22-29

## Revendications

1. Procédé pour pronostiquer, *in vitro,* dans un échantillon sanguin, la probabilité pour un patient d'évoluer vers une dengue sévère, dans lequel :
a)on détermine la quantité d'au moins un marqueur qui est la Leucine-riche alpha-2 glycoprotéine dans ledit échantillon sanguin,
b)on compare la quantité de la leucine-riche alpha-2 glycoprotéine déterminée dans l'étape a) avec une quantité de référence dudit marqueur obtenue à partir d'un groupe d'individus qui ont été diagnostiqués comme étant atteints d'une dengue non sévère,
dans lequel si la quantité de la leucine-riche alpha-2 glycoprotéine déterminée dans l'étape a) est supérieure à la quantité de référence établie dans l'étape b), on pronostique que le patient va évoluer vers une dengue sévère.

2. Procédé selon la revendication 1, dans lequel dans l'étape a) on détermine en plus la quantité d'au moins un autre marqueur choisi parmi , la Vitamine D Binding-Protéine et la Ferritine dans l'échantillon sanguin et dans l'étape b) on compare la quantité dudit autre marqueur de l'étape a) avec une quantité de référence dudit autre marqueur obtenue à partir d'un groupe d'individus qui ont été diagnostiqués comme étant atteints d'une dengue non sévère et si la quantité dudit au moins autre marqueur déterminée dans l'étape a) est supérieure à la quantité de référence établie dans l'étape b), on pronostique que le patient va évoluer vers une dengue sévère.

3. Procédé selon la revendication 1, dans lequel dans l'étape a) on détermine en plus la quantité d'au moins deux autres marqueurs choisi parmi la Vitamine D Binding-Protéine et la Ferritine dans l'échantillon sanguin et dans l'étape b) on compare la quantité de chacun des deux autres marqueurs de l'étape a) avec une quantité de référence pour chacun desdits autres marqueurs obtenue à partir d'un groupe d'individus qui ont été diagnostiqués comme étant atteints d'une dengue non sévère et si la quantité de chacun des autres marqueurs déterminée dans l'étape a) est supérieure à la quantité de référence de chacun des marqueurs établie dans l'étape b), on pronostique que le patient va évoluer vers une dengue sévère.

4. Utilisation de la Leucine-riche alpha-2 glycoprotéine comme marqueur pour le pronostic *in vitro* d'une dengue sévère chez un patient.

5. Utilisation selon la revendication 4, de la Vitamine D Binding Protéine ou de la Ferritine comme marqueur supplémentaire pour le pronostic *in vitro* d'une dengue sévère chez un patient.

6. Utilisation selon la revendication 4 de la Vitamine D Binding Protéine et de la Ferritine comme marqueurs supplémentaires pour le pronostic *in vitro* d'une dengue sévère chez un patient.

7. Kit pour le pronostic *in vitro* d'une dengue sévère comprenant un partenaire de liaison de la Leucine-riche alpha-2 glycoprotéine, un partenaire de liaison de la Vitamine D Binding Protéine et un partenaire de liaison de la Ferritine.

8. Kit selon la revendication 7, comprenant de plus au moins un partenaire de liaison d'une protéine du virus de la dengue choisie parmi la protéine NS1, la protéine d'enveloppe et la protéine prM.

## Patentansprüche

1. Verfahren für eine *in-vitro*-Prognose, in einer Blutprobe, der Wahrscheinlichkeit, dass sich bei einem Patienten ein schweres Denguefieber entwickelt, wobei
a) in der Blutprobe die Menge mindestens eines Markers bestimmt wird, bei welchem es sich das leucinreiche alpha-2-Glycoprotein handelt,
b) die Menge an leucinreichem alpha-2-Glycoprotein, wie sie in Schritt a) bestimmt wurde, mit einer Bezugsmenge ebendieses Markers verglichen wird, die ausgehend von einer Gruppe von Personen erhalten wurde, bei welchen das Vorliegen einer nicht-schweren Form des Denguefiebers diagnostiziert wurde,
wobei, wenn die Menge an leucinreichem alpha-2-Glycoprotein, wie sie in Schritt a) bestimmt wurde, größer als die Bezugsmenge ist, wie sie in Schritt b) erhalten wurde, die Prognose gestellt wird, dass sich bei dem Patienten ein schweres Denguefieber entwickeln wird.

2. Verfahren nach Anspruch 1, wobei in Schritt a). in der Blutprobe zusätzlich die Menge an mindestens einem weiteren Marker bestimmt wird, der aus dem Vitamin-D-bindenden Protein und Ferritin ausgewählt ist, und in Schritt b) die Menge des weiteren Markers des Schritts a) mit einer Bezugsmenge ebendieses Markers verglichen wird, wie sie ausgehend von einer Gruppe von Personen erhalten wurde, bei welchen das Vorliegen einer nicht-schweren Form des Denguefiebers diagnostiziert wurde, und wobei, wenn die Menge des mindestens einen weiteren Markers, wie sie in Schritt a) bestimmt wurde, größer als die Bezugsmenge ist, wie sie in Schritt b) erhalten wurde, die Prognose gestellt wird, dass sich bei dem Patienten ein schweres Denguefieber entwickeln wird.

3. Verfahren nach Anspruch 1, wobei in Schritt a) in der Blutprobe zusätzlich die Menge an mindestens zwei weiteren Markern bestimmt wird, die aus dem Vitamin-D-bindenden Protein und Ferritin ausgewählt sind, und in Schritt b) die Menge jedes der beiden weiteren Marker des Schritts a) jeweils mit einer Bezugsmenge für diese weiteren Marker verglichen wird, wie sie ausgehend von einer Gruppe von Personen erhalten wurde, bei welchen das Vorliegen einer nicht-schweren Form des Denguefiebers diagnostiziert wurde, und wobei, wenn die Menge jedes der weiteren Marker, wie sie in Schritt a) bestimmt wurde, größer als die jeweilige Bezugsmenge der weiteren Marker ist, wie sie in Schritt b) erhalten wurde, die Prognose gestellt wird, dass sich bei dem Patienten ein schweres Denguefieber entwickeln wird.

4. Verwendung des leucinreichen alpha-2-Glycoproteins als Marker für die *in-vitro*-Prognose eines schweren Denguefiebers bei einem Patienten.

5. Verwendung nach Anspruch 4 des Vitamin-D-bindenden Proteins oder von Ferritin als zusätzlicher Marker für die *in-vitro*-Prognose eines schweren Denguefiebers bei einem Patienten.

6. Verwendung nach Anspruch 4 des Vitamin-D-bindenden Proteins und von Ferritin als zusätzliche Marker für die *in-vitro*-Prognose eines schweren Denguefiebers bei einem Patienten.

7. Anwendungseinheit zur *in-vitro*-Prognose eines schweren Denguefiebers, wobei diese einen Bindungspartner des leucinreichen alpha-2-Glycoproteins, einen Bindungspartner des Vitamin-D-bindenden Proteins und einen Bindungspartner von Ferritin umfasst.

8. Anwendungseinheit nach Anspruch 7, wobei diese darüber hinaus mindestens einen Bindungspartner eines Proteins des Denguefiebervirus umfasst, welches aus dem Protein NS1, dem Hüllprotein und dem Protein prM ausgewählt ist.

## Claims

1. A method for the *in vitro* prediction of the probability of a patient developing severe dengue, based on a blood sample, which involves:
a) determining the quantity of at least one marker which is leucine-rich alpha-2 glycoprotein in said blood sample,
b) comparing the quantity of leucine-rich alpha-2 glycoprotein determined in step a) with a reference quantity of said marker obtained from a group of individuals who have been diagnosed with non-severe dengue,
wherein, if the quantity of leucine-rich alpha-2 glycoprotein determined in step a) is greater than the reference quantity established in step b), it is predicted that the patient will develop severe dengue.

2. The method as claimed in claim 1, wherein, in step a), the quantity of at least one other marker chosen from vitamin D-binding protein and ferritin is determined in the blood sample and, in step b), the quantity of said other marker of step a) is compared with a reference quantity of said other marker obtained from a group of individuals who have been diagnosed with non-severe dengue and, if the quantity of said at least one other marker determined in step a) is greater than the reference quantity established in step b), it is predicted that the patient will develop severe dengue.

3. The method as claimed in claim 1, wherein, in step a), the quantity of at least two other markers chosen from vitamin D-binding protein and ferritin is determined in the blood sample and, in step b), the quantity of each of the two other markers of step a) is compared with a reference quantity for each of said other markers obtained from a group of individuals who have been diagnosed with non-severe dengue and, if the quantity of each of the other markers determined in step a) is greater than the reference quantity of each of the markers established in step b), it is predicted that the patient will develop severe dengue.

4. The use of leucine-rich alpha-2 glycoprotein as a marker for the *in vitro* prediction of severe dengue in a patient.

5. The use as claimed in claim 4, of vitamin D-binding protein or of ferritin as a marker for the *in vitro* prediction of severe dengue in a patient.

6. The use as claimed in claim 4, of vitamin D-binding protein and of ferritin as markers for the *in vitro* prediction of severe dengue in a patient.

7. A kit for the *in vitro* prediction of severe dengue, comprising a binding partner of leucine-rich alpha-2 glycoprotein, a binding partner of vitamin D-binding protein and a binding partner of ferritin.

8. The kit as claimed in claim 7, also comprising at least one binding partner of a dengue virus protein chosen from the NS1 protein, the envelope protein and the prM protein.
